(19) Europäisches Patentamt
European Patent Office
Office européen des brevets

(11) EP 3 147 290 A1

(12) EUROPÄISCHE PATENTANMELDUNG

(43) Veröffentlichungstag:
**29.03.2017 Patentblatt 2017/13**

(51) Int Cl.:
***C07F 9/6574*** *(2006.01)* ***C07C 45/50*** *(2006.01)*

(21) Anmeldenummer: **16186615.7**

(22) Anmeldetag: **31.08.2016**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(30) Priorität: **22.09.2015 EP 15186168**

(71) Anmelder: **Evonik Degussa GmbH**
**45128 Essen (DE)**

(72) Erfinder:
- **Börner, Armin**
  **18059 Rostock (DE)**
- **Dyballa, Katrin Marie**
  **45657 Recklinghausen (DE)**
- **Franke, Robert**
  **45772 Marl (DE)**
- **Fridag, Dirk**
  **45721 Haltern am See (DE)**
- **Geilen, Frank**
  **45721 Haltern am See (DE)**
- **Hess, Dieter**
  **45770 Marl (DE)**
- **Häger, Harald**
  **59348 Lüdinghausen (DE)**
- **Selent, Detlef**
  **18059 Rostock (DE)**

Bemerkungen:
Geänderte Patentansprüche gemäss Regel 137(2) EPÜ.

# (54) MONOPHOSPHITE, DIE EIN BINOL-DERIVAT AUFWEISEN

(57) Die Erfindung betrifft Monophosphite, die ein 1,1'-Bi-2-naphthol (BINOL) aufweisen, sowie deren Verwendung als Liganden für Hydroformylierungskatalysatoren.

EP 3 147 290 A1

## Beschreibung

Die Erfindung betrifft Monophosphite, die ein Derivat von 1,1'-Bi-2-naphthol (BINOL) aufweisen, sowie deren Verwendung als Liganden für Hydroformylierungskatalysatoren.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigen Phosphor PIII. Eine gute Übersicht über den Stand der Hydroformylierung von Olefinen findet sich in B. Cornils, W. A. Herrmann, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI:10.1021/cr3001803.

Jede katalytisch aktive Zusammensetzung hat ihre spezifischen Vorzüge. Je nach Einsatzstoff und Zielprodukt kommen daher unterschiedliche katalytisch aktive Zusammensetzungen zum Einsatz.

Die Hydroformylierung führt, außer im Fall von Ethylen als Edukt, zu einer Mischung isomerer Produkte, nämlich n-Aldehyden (lineare Aldehyde) und iso-Aldehyden (verzweigte Aldehyde). Neben der Reaktionsgeschwindigkeit ist somit auch die Selektivität bei der Bildung von n- bzw. iso-Produkten ein wichtiger Parameter der Hydroformylierungsreaktion.

Die einfache Verfügbarkeit und damit verbunden die gute Möglichkeit eines großtechnischen Einsatzes ist ein wichtiges Kriterium, da der Herstellungsaufwand und damit verbunden die anfallenden Kosten nur so hoch sein dürfen, dass die Rentabilität des Gesamtprozesses weiterhin gewährleistet ist.

Rhodium-Monophosphit-Komplexe in katalytisch aktiven Zusammensetzungen sind geeignet für die Hydroformylierung von verzweigten Olefinen mit innenständigen Doppelbindungen.

Seit den 1970er Jahren ist die Verwendung von so genannten "bulky phosphites" in der Hydroformylierungbeschrieben (siehe u.a. van Leeuwen et. al, Journal of Catalysis, 2013, 298, 198-205). Diese zeichnen sich durch eine gute Aktivität aus, jedoch ist die n/i-Selektivität für endständig oxierte Verbindungen gering und verbesserungswürdig.

Aus EP 0 155 508 A1 ist die Verwendung von bisarylensubstituierten Monophosphiten bei der rhodiumkatalysierten Hydroformylierung von sterisch gehinderten Olefinen, z. B. Isobuten, bekannt. Hierbei werden jedoch z.T. sehr hohe Rhodiumkonzentrationen verwendet (u.a. 250 ppm), was in Anbetracht des derzeitigen Rhodiumpreises für ein großtechnisches Verfahren inakzeptabel ist und verbessert werden muss.

Für Hydroformylierungsreaktionen ist derzeit Tris(2,4-di-tert-butylphenyl)phosphit (TDTBPP) einer der leistungsstärksten und kommerziell verfügbaren Monophosphitliganden, welcher unter dem Handelsnamen Alkanox 240 erhältlich ist (siehe auch R. Franke, D. Selent, A. Börner, "Applied Hydroformylation", Chem. Rev., 2012, 112, S.5681 Kapitel 3.4.2).

Der vorliegenden Erfindung lag vor diesem Hintergrund die Aufgabe zugrunde, neue Liganden für die Hydroformylierung bereitzustellen, welche eine hohe Ausbeute bei gleichzeitig hoher n-Selektivität gewährleisten.

Die Aufgabe wird gelöst durch eine Verbindung gemäß Formel (I):

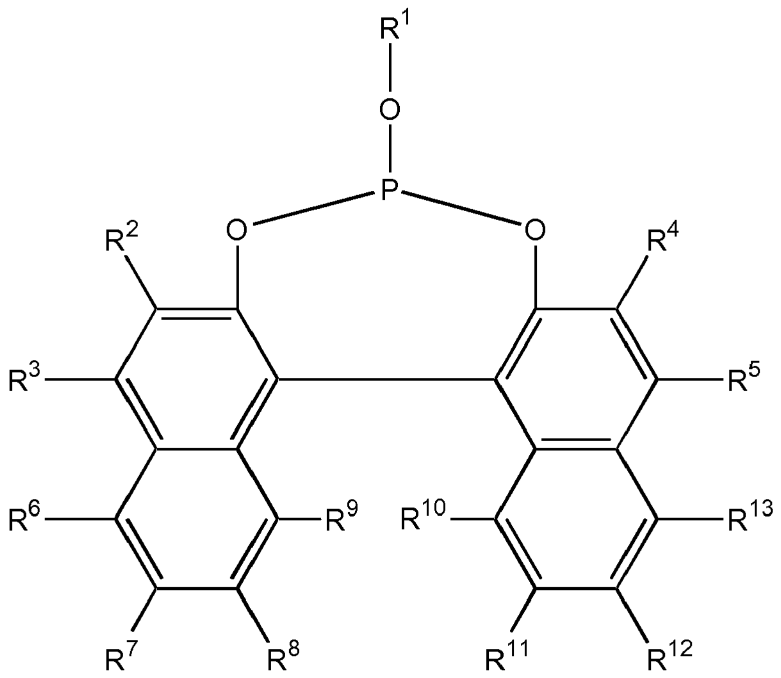

(I)

wobei $R^1$ Anthracenyl oder Diphenylphenyl darstellt;

$R^2$ bis $R^{13}$ unabhängig voneinander ausgewählt sein können aus -H, -($C_6$-$C_{20}$)-Aryl, -($C_1$-$C_{12}$)-Alkyl, -($C_3$-$C_{12}$)-Cycloalkyl, -($C_3$-$C_{12}$)-Heterocycloalkyl, -O-($C_1$-$C_{12}$)-Alkyl, -O-($C_3$-$C_{12}$)-Cycloalkyl, -N-[($C_1$-$C_{12}$)-Alkyl]$_2$, -COOH, -OH, -$SO_3H$, -$NH_2$, und Halogen.

Die erfindungsgemäßen Phosphite gemäß Formel (I), welche mit 1,1'-Bi-2-naphthol substituiert sind, eignen sich in besonderer Weise als Liganden für Hydroformylierungskatalysatoren. Unter Verwendung dieser Liganden lassen sich hohe Ausbeuten sowie eine hohe Selektivität bei der Herstellung von n-Aldehyden aus endständigen Olefinen erzielen. Insbesondere hat sich dies bei der Hydroformylierung von n-Octen gezeigt. Diese Vorteile lassen sich außerdem bei einem relativ geringen Verhältnis von Ligand zu katalytisch aktivem Edelmetall, z.B. Rh, erzielen.

Bei die Binaphthol bzw. Octahydrodinaphtholgruppe kann eine S- oder R-Konfiguration aufweisen und auch als Racemat vorliegen.

Die beiden Naphtholgruppen bzw. teilhydrierten Naphtholgruppen mit den Substituenten $R^2$ bis $R^{21}$ können symmetrisch oder asymmetrisch substituiert sein. In einer bevorzugten Ausführungsform sind sie symmetrisch substituiert.

Im Rahmen der Erfindung umfasst der Ausdruck -($C_1$-$C_{12}$)-Alkyl geradkettige und verzweigte Alkylgruppen mit 1 bis 12 Kohlenstoffatomen. Vorzugsweise handelt es sich dabei um geradkettige oder verzweigte -($C_1$-$C_8$)-Alkyl- und ganz bevorzugt um geradkettige oder verzweigte -($C_1$-$C_6$)-Alkylgruppen. Beispiele für -($C_1$-$C_{12}$)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Die Erläuterungen zum Ausdruck -($C_1$-$C_{12}$)-Alkyl gelten auch für die Alkylgruppen in -O-($C_1$-$C_{12}$)-Alkyl, -S-($C_1$-$C_{12}$)-Alkyl, -COO-($C_1$-$C_{12}$)-Alkyl, -CONH-($C_1$-$C_{12}$)-Alkyl, -CO-($C_1$-$C_{12}$)-Alkyl, und -N-[($C_1$-$C_{12}$)-Alkyl]$_2$.

Der Ausdruck -($C_3$-$C_{12}$)-Cycloalkyl umfasst im Sinne der vorliegenden Erfindung zyklische Kohlenwasserstoffreste mit 3 bis 12, insbesondere 5 bis 12 Kohlenstoffatomen. Insbesondere handelt es sich dabei um mono-, bi-odertricyclische Kohlenwasserstoffreste. Dazu zählen Cyclopropyl, Cyclobutyl, Cyclopentyl, Cyclohexyl, Cycloheptyl, Cyclooctyl, Cyclododecyl, Cyclopentadecyl, Norbonyl oder Adamantyl. Unter den Begriff -($C_3$-$C_{12}$)-Cycloalkyl fallen auch alkylsubstituierte Cycloalkylgruppen mit insgesamt 3 bis 12 Kohlenstoffatomen, wie beispielsweise Menthyl.

Der Ausdruck -($C_3$-$C_{12}$)-Heterocycloalkyl umfasst im Sinne der vorliegenden Erfindung nichtaromatische, gesättigte oder teilweise ungesättigte cycloaliphatische Gruppen mit 3 bis 12, insbesondere 5 bis 12, Kohlenstoffatomen. Die -($C_3$-$C_{12}$)-Heterocycloalkylgruppen weisen vorzugsweise 3 bis 8, besonders bevorzugt 5 oder 6, Ringatome auf. In den Heterocycloalkylgruppen sind im Unterschied zu den Cycloalkylgruppen 1, 2, 3 oder 4 der Ringkohlenstoffatome

durch Heteroatome oder heteroatomhaltige Gruppen ersetzt. Die Heteroatome oder die heteroatomhaltige Gruppen sind vorzugsweise ausgewählt unter-O-, -S-, -N-, -N(=O)-, -C(=O)- oder -S(=O)-. Beispiele für -($C_3$-$C_{12}$)-Heterocycloalkylgruppen Tetrahydrothiophenyl, Tetrahydrofuryl, Tetrahydropyranyl und Dioxanyl.

Der Ausdruck -($C_6$-$C_{20}$)-Aryl umfasst im Sinne der vorliegenden Erfindung mono- oder polycyclische aromatische Kohlenwasserstoffreste mit 6 bis 20 Kohlenstoffatomen. Geeignete Arylgruppen sind beispielsweise Phenyl, Naphthyl, Indenyl, Fluroenyl, Anthracenyl, Phenanthrenyl, Naphthacenyl, Chrysenyl, Pyrenyl, und Coronenyl.

Bei den genannten Halogen-Substituenten handelt es sich bevorzugt um Fluor oder Chlor oder lod, bevorzugt Chlor oder Fluor.

Die Gruppe $R^1$ kann unsubstituiert oder mit einer oder mehrerer der erwähnten Gruppen substituiert sein.

In einer weiteren bevorzugten Ausführungsform stellt $R^1$ Anthracenyl oder Diphenylphenyl dar. Hierbei kommt als Diphenylphenyl vor allem 3,5-Diphenylphenyl in Betracht.

In einer Ausführungsform sind $R^2$ bis $R^{13}$ unabhängig voneinander ausgewählt aus -H, -($C_1$-$C_{12}$)-Alkyl, und -O-($C_1$-$C_{12}$)-Alkyl, bevorzugt aus -H, -($C_1$-$C_6$)-Alkyl, und -O-($C_1$-$C_6$)-Alkyl.

In einer Ausführungsform stellen $R^2$ bis $R^{13}$ jeweils -H dar.

In einer bevorzugten Ausführungsform stellt $R^1$ Anthracenyl oder Diphenylphenyl dar und stellen $R^2$ bis $R^{13}$ jeweils -H dar.

In einer weiteren Ausführungsform weist die erfindungsgemäße Verbindung eine der folgenden Strukturen (1), (2), oder (4) auf:

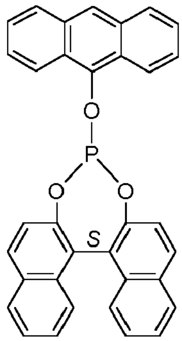

(**1**)

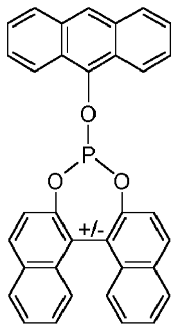

(**2**)

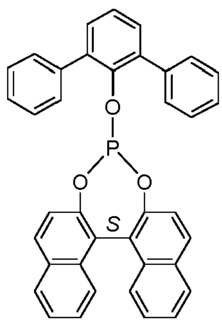

(**4**)

Neben den genannten Verbindungen betrifft die Erfindung auch einen Komplex, welcher die genannte Verbindung und ein Metallatom ausgewählt aus Rh, Ru, Co und Ir umfasst. In einer bevorzugten Ausführungsform ist das

Metall Rh.

Die Herstellung derartiger Edelmetallkomplexe ist aus dem Stand der Technik bekannt. Siehe hierzu R. Franke, D. Selent, A. Börner, "Applied Hydroformylation", Chem. Rev., 2012, DOI:10.1021/cr3001803; S. 5688 Schema 12 "General Method forthe Preparation of a P-Modified Rh precatalyst" und darin zitierte Literaturstellen sowie P. W. N. M. van Leeuwen, in Rhodium Catalyzed Hydroformylation, P. W. N. M. van Leeuwen, C. Claver (Hrsg.), Kluwer, Dordrecht, 2000 unter anderem S. 48 ff, S.233 ff. und darin zitierte Literaturstellen sowie K.D. Wiese und D. Obst in Top. Organomet. Chem. 2006, 18, 1-13; Springer Verlag Berlin Heidelberg 2006 S. 6 ff sowie darin zitierte Literaturstellen.

Des Weiteren betrifft die Erfindung die Verwendung der Verbindung zur Katalyse einer Hydroformylierungsreaktion. Dabei wird die erfindungsgemäße Verbindung bevorzugt als Ligand in einem erfindungsgemäßen Ligand-Metall-Komplex eingesetzt. Ebenso betrifft die Erfindung die Verwendung des beschriebenen Komplexes zur Katalyse einer Hydroformylierungsreaktion. Besonders bevorzugt ist hierbei die Hydroformylierung endständiger Olefine zu Aldehyden.

Die Erfindung betrifft ebenfalls ein Verfahren, bei dem die erfindungsgemäße Verbindung als Ligand in einem Ligand-Metall-Komplex zur Umsetzung eines Olefins zu einem Aldehyd eingesetzt wird.

Das erfindungsgemäße Verfahren umfasst die Verfahrensschritte:

a) Vorlegen eines Olefins,
b) Zugabe eines erfindungsgemäßen Komplexes oder einer erfindungsgemäßen Verbindung und einer Substanz, welche ein Metallatom ausgewählt aus Rh, Ru, Co und Ir aufweist,
c) Zuführen von Wasserstoff und Kohlenstoffmonoxid,
d) Erwärmen des Reaktionsgemisches, wobei das Olefin zu einem Aldehyd umgesetzt wird.

Hierbei können die Verfahrensschritte a) bis c) in beliebiger Reihenfolge erfolgen.

In einer bevorzugten Variante des Verfahrens ist das Metallatom Rh.

Es kann hierbei auch ein Überschuss an Liganden verwendet werden und nicht zwangsläufig jeder Ligand liegt gebunden in Form eines Ligand-Metall-Komplexes vor, sondern ist als freier Ligand im Reaktionsgemisch enthalten. Vorzugsweise liegt das molare Verhältnis der erfindungsgemäßen Verbindung zum Metallatom im Bereich von 10:1 zu 1:1, bevorzugt 8:1 zu 1:1, besonders bevorzugt 6:1 zu 2:1.

Die Reaktion wird bevorzugt bei einer Temperatur von 80 °C bis 200 °C und einem Druck von 1 bar bis 300 bar durchgeführt. Besonders bevorzugt sind eine Temperatur von 100 °C bis 160 °C und ein Druck von 5 bar bis 60 bar. Besonders bevorzugt sind eine Temperatur von 110 °C bis 130 °C und ein Druck von 10 bar bis 30 bar.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1- oder 2-Penten, 2-Methyl-1-buten, 2-Methyl-2-buten, 3-Methyl-1-buten, 1-, 2- oder 3-Hexen, das bei der Dimerisierung von Propen anfallende $C_6$-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende $C_8$-Olefingemisch (Dibuten), Nonene, 2-oder 3-Methyloctene, das bei der Trimerisierung von Propen anfallende $C_9$-Olefingemisch (Tripropen), Decene, 2-Ethyl-1-octen, Dodecene, das bei der Tetramerisierung oder der Trimerisierung von Butenen anfallende $C_{12}$-Olefingemisch (Tetrapropen oder Tributen), Tetradecene, Hexadecene, das bei der Tetramerisierung von Butenen anfallende $C_{16}$-Olefingemisch (Tetrabutan) sowie durch Cooligomerisierung von Olefinen mit unterschiedlicher Anzahl von Kohlenstoffatomen (bevorzugt 2 bis 4) hergestellte Olefingemische. Bevorzugt handelt es sich um Monoolefine mit einer endständigen C-C-Doppelbindung.

## Beispiele

Die Erfindung wird anhand der folgenden Ausführungsbeispiele erläutert.

## Allgemeine Arbeitsvorschriften

Alle präparativen Arbeiten erfolgten unter Anwendung der Schlenk-Technik mit Argon als Schutzgas. Toluol und Tetrahydrofuran wurden mit einem Pure Solv MD-7 System gereinigt und bis zur Verwendung unter Argon aufbewahrt. Triethylamin wurde vordem Einsatz unter Argon von Natriumketyl destilliert. Phosphortrichlorid (Aldrich) wurde vor dem Einsatz unter Argon destilliert. Alle präparativen Arbeiten erfolgten in ausgeheizten Gefäßen. Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten, die halbautomatische Säulenchromatografie an einem Teledyne Isco Combiflash Rf+.

Ligand 1

4-(Anthracen-9-yloxy)-S-dinaphtho[2.1-*d*:1',2'-*f*][1,3,2]dioxaphosphepin

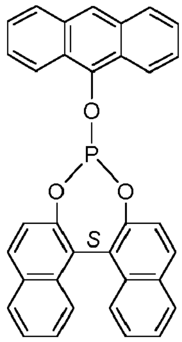

(**1**)

Eine bei -20 °C gerührte Suspension von Anthron (0,447 g; 2,30 mmol) in THF (5 ml) wird tropfenweise mit einer 0,32 M Lösung von *n*-Butyllithium in Heptan (7,2 ml; 2,30 mmol) versetzt. Man lässt auf Raumtemperatur kommen und tropft dann unter Rühren eine Lösung von 4-Chlor-*S*-dinaphtho[2,1-*d*:1',2'-*f*][1,3,2]dioxaphosphepin (0,807 g; 2,30 mmol) in THF (6 ml) zu. Es wird über Nacht gerührt und filtriert. Man entfernt die flüchtigen Bestandteile des Filtrates im Vakuum, nimmt den erhaltenen gelben Feststoff in Toluol (10 ml) auf, filtriert über eine mit Kieselgel beschichtete G4-Fritte und engt das Filtrat im Vakuum ein. Der erhaltene Feststoff wird 3 h bei 50°C/0,1 mbar getrocknet. Die säulenchromatografische Aufarbeitung (Hexan/$CH_2Cl_2$, Gradient Hexan 100 → 0 %; $R_f$= 0,5 für das 1:1-Gemisch der Eluenten) ergibt 0,55 g (1,08 mmol; 47%) an reinem Produkt.

Elementaranalyse (ber. für $C_{34}H_{21}O_3P$= 508,511 g/mol): C 80,80 (80,31); H 3,89 (4,16); P 6,02 (6,09)%. ESI-TOF/HRMS: m/e 509,12979 $(M+H)^+$.

$^{31}P$-NMR ($CD_2Cl_2$): 149,7 (s) ppm.

$^1H$-NMR ($CD_2Cl_2$): 7,37-7,42 (m, 2H); 7,51-7,65 (m, 9H); 7,87 (d, $J_{HH}$= 8,9 Hz, 1 H); 8,02-8,14 (m, 6H); 8,40 (s, 1 H); 8,57 (d, $J_{HH}$= 8,9 Hz, 2H) ppm.

$^{13}C$-NMR ($CD_2Cl_2$): 122,1; 122,4; 123,0; 123,6; 123,8; 124,7; 125,0; 125,7; 126,1; 126,5; 127,0; 127,3; 128,6; 128,9; 130,6; 131,1; 131,9; 132,3; 132,6; 133,1; 133,4; 143,5 (d, $J_{cp}$= 6,1 Hz); 147,4 (d, $J_{cp}$= 2,7 Hz); 148,3 (d, $J_{cp}$= 5,2 Hz) ppm.

**Ligand 2**

*rac*-4-(Anthracen-9-yloxy)dinaphtho[2,1-*d*:1',2'-*f*][1,3,2]dioxaphosphepin

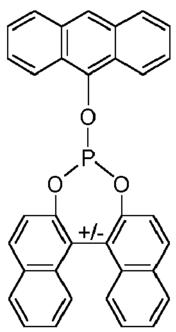

(**2**)

Eine bei -20 °C gerührte Suspension von Anthron (0,447 g; 2,30 mmol) in THF (6 ml) wird tropfenweise mit einer 1,6 M Lösung von *n*-Butyllithium in Heptan (1,44 ml; 2,30 mmol) versetzt. Man lässt auf Raumtemperatur kommen und tropft dann unter Rühren eine Lösung von *rac*-4-Chlor-dinaphtho[2,1-*d*:1',2'-*f*][1,3,2]dioxaphosphepin (0,86 g; 2,45 mmol) in THF (8 ml) zu. Es wird über Nacht gerührt und filtriert. Man entfernt die flüchtigen Bestandteile des Filtrates

im Vakuum, nimmt den erhaltenen gelben Feststoff in Toluol (12 ml) auf, filtriert über eine mit Kieselgel beschichtete G4-Fritte und engt das Filtrat im Vakuum ein. Der erhaltene Feststoff wird 2 h bei 50°C/0,1 mbar getrocknet. Die säulenchromatografische Aufarbeitung (Heptan/$CH_2Cl_2$ = 4:1, $R_f$= 0,25 für das 3:1-Gemisch der Eluenten) ergibt 0,38 g (1,08 mmol; 47%).

Elementaranalyse (ber. für $C_{34}H_{21}O_3P$= 516,574 g/mol): C 80,19 (80,31); H 4,22 (4,16) %. $^{31}$P-NMR ($CD_2Cl_2$): 149,0 (s) ppm.

$^1$H-NMR ($CD_2Cl_2$): 7,22-7,28 (m, 2H); 7,33-7,47 (m, 8H); 7,49 (d, $J_{HH}$= 8,7 Hz, 1H); 7,70 (d, $J_{HH}$= 8,7 Hz, 1 H); 7,89-8,00 (m, 6H); 8,26 (s, br, 1 H); 8,38-8,40 (m, 2H) ppm.

$^{13}$C-NMR ($CD_2Cl_2$): 122,1; 122,3; 122,9; 123,4; 123,7; 124,7; 125,0; 125,6; 125,8; 126,1; 126,4; 126,8; 126,9; 127,2; 128,6; 128,9; 130,5; 131,0; 131,8; 132,2; 132,5; 133,0; 133,2; 143,4 (d, $J_{cp}$= 5,6 Hz); 147,4 (d, $J_{cp}$= 3,0 Hz); 148,3 (d, $J_{cp}$= 5,5 Hz) ppm.

**Ligand 3 (Vergleichsbeispiel)**

4-(((1*R*.2*S*.5*R*)-2-isopropyl-5-methylcyclohexyl)oxy)-*S*-dinaphtho[2.1-*d*:1',2'-*f*][1,3,2]dioxaphosphepin

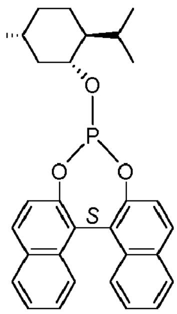

(**3**)

Zu einer Lösung von (-)-Menthol (0,368 g; 2,356 mmol) in THF (7 ml) wird bei -20 °C unter Rühren eine 0,32 M Lösung von *n*-Butyllithium in Heptan (7,36 ml; 2,365 mmol) gegeben. Man rührt 20 min, lässt auf Raumtemperatur kommen und tropft die erhaltene Lösung unter Rühren zu einer auf -20 °C gekühlten Lösung von 4-Chlor-*S*-dinaphtho[2,1-*d*:1',2'-*f*] [1,3,2]dioxaphosphepin (0,843 g; 2,403 mmol) in THF (7 ml). Es wird 20 min bei -20 °C und anschließend über Nacht bei Raumtemperatur gerührt. Es wird filtriert, das Filtrat im Vakuum zur Trockne eingeengt und der Rückstand 2 h bei 50°C/0,1 mbar getrocknet. Der erhaltene Feststoff wird zunächst säulenchromatografisch aufgearbeitet (Hexan/Toluol, 1:1, $R_f$= 0,55) und das per SC erhaltene Produkt bei Raumtemperatur 1 Stunde mit Hexan (12 ml) verrührt. Abtrennen der flüssigen Phase und Trocknen des verbleibenden Feststoffes für 3 h bei 50°C/0,1 mbar ergibt 0,687 g (1,461 mmol; 61%).

Elementaranalyse (ber. für $C_{30}H_{31}O_3P$ = 470,546 g/mol): C 76,36 (76,58); H 6,51 (6,64); P 6,51 (6,58)%. ESI-TOF/HRMS: m/e 509,16616 ($M$+K)$^+$.

$^{31}$P-NMR ($CD_2Cl_2$): 153,4 (s) ppm.

$^1$H-NMR ($CD_2Cl_2$): 0,85-1,35 (mehrfache Signalüberlappung, 12H); 1,38-1,55 (m, 2H), 1,72 (m, 2H); 2,16 (m, 1H); 2,39 (m, 1H); 4,19 (m, 1H); 7,28-7,35 (m, 2H); 7,37 (s, br, 1H); 7,39 (s, br, 1 H); 7,44-7,61 (m, 4H); 7,08-8,05 (m, 4H) ppm.

$^{13}$C-NMR ($CD_2Cl_2$): 16,0; 21,3; 22,2; 23,3; 25,9; 32,2; 34,5; 44,6; 49,0; 77,4 (d, $J_{cp}$= 16,0 Hz); 122,3; 122,5; 123,3; 124,6; 125,3; 125,6; 126,6; 126,7; 127,2; 128,6; 128,8; 128,9; 129,4; 130,1; 130,8; 131,6; 132,0; 133,0; 133,2; 148,2 (d, $J_{cp}$= 2,2 Hz); 148,6 (d, $J_{cp}$= 5,3 Hz) ppm.

**Ligand 4**

4-([1,1':3',1"-Terphenyl]-2'-yloxy)-*S*-dinaphtho[2,1-*d*:1',2'-*f*][1,3,2]dioxaphosphepin

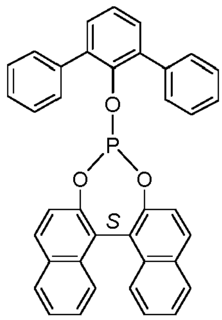

(**4**)

Zu einer Lösung von 2,6-Diphenylphenol (0,568 g; 2,30 mmol) in THF (7 ml) wird bei -20 °C unter Rühren eine 0,32 M Lösung von *n*-Butyllithium in Heptan (7,2 ml; 2,30 mmol) gegeben. Man rührt 20 min, lässt auf Raumtemperatur kommen und tropft die erhaltene Lösung unter Rühren zu einer auf -20 °C gekühlten Lösung von 4-Chlor-S-dinaphtho[2,1-*d*:1',2'-*f*][1,3,2]dioxaphosphepin (0,849 g; 2,42 mmol) in THF (8 ml). Es wird 20 min bei - 20°C und anschließend über Nacht bei Raumtemperatur gerührt. Man entfernt das Lösungsmittel im Vakuum, nimmt den Rückstand in Toluol (14 ml) auf, filtriert, engt zur Trockne ein und trocknet den erhaltenen Feststoff für 1 h bei 50°C/0,1 mbar. Die säulenchromatografische Aufarbeitung (Hexan/Toluol, Gradient Hexan 100 → 0 %; $R_f$= 0,6 für das 1:2-Gemisch der Eluenten) ergibt nach Entfernen der Lösungsmittel und Trocknen für 3 h bei 50°C/0,1 mbar 1,066 g (1,90 mmol; 82%) an Produkt, welches nach $^1$H-NMR-spektroskopischem Befund Spuren von 2,6-Diphenylphenol und Toluol enthält. Elementaranalyse (ber. für $C_{38}H_{25}O_3P$= 560,586 g/mol): C 81,30 (81,42); H 4,71 (4,49); P 5,56 (5,53)%.

ESI-TOF/HRMS: m/e 561,16060 ($M$+H)$^+$.

$^{31}$P-NMR ($CD_2Cl_2$): 146,4 (s) ppm. $^1$H-NMR ($CD_2Cl_2$): 5,97 (d, $J_{HH}$= 8,8 Hz, 1H); 6,86 (d, $J_{HH}$= 8,8 Hz, 1 H); 7,22-7,32 (m, 4 H); 7,34-7,39 (m, 1 H); 7,42-7,50 (m, 4H); 7,54-7,64 (m, 10H); 7,71 (d, $J_{HH}$= 8,8 Hz, 1 H); 7,88-7,98 (m, 3H) ppm.

$^{13}$C-NMR ($CD_2Cl_2$): 121,9; 122,2; 122,4; 124,6; 125,1; 125,2; 125,5; 126,4; 126,6; 127,0; 127,1; 127,); 128,6; 128,7; 128,9; 129,2; 129,4; 130,1; 130,6; 131,0; 131,2; 131,4; 131,9; 132,5; 132,9; 136,4; 138,8; 146,4 (d, $J_{cp}$= 8,3 Hz); 147,0; 148,2 (d, $J_{cp}$= 4,8 Hz) ppm.

**Ligand 5 (Vergleichsbeispiel)**

6-([1,1':3',1"-Terphenyl]-2'-yloxy)-4,8-di-*tert*-butyl-2,10-dimethoxydibenzo[d,*f*][1,3,2]dioxaphosphepin.

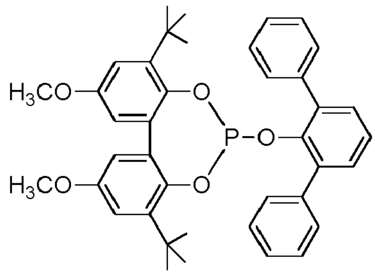

(**5**)

Eine Lösung von 2,6-Diphenylphenol (0,411 g; 1,65 mmol) in Toluol (8 ml) wurde mit Triethylamin (1,529 g; 15,11 mmol) versetzt und die erhaltene Mischung tropfenweise bei 0 °C zu einer Lösung von 4,8-Di-*tert*-butyl-6-chloro-2,10-dimethoxydibenzo [*d*,*f*][1,3,2]dioxaphosphepin (0,697 g; 1,65 mmol) in Toluol (6 ml) gegeben. Die Reaktionsmischung wurde über Nacht bei Raumtemperatur und für 5 h bei 70 °C gerührt. Dann wurde die Mischung filtriert und das Filtrat im Vakuum bis zur Trockne eingeengt. Der erhaltene Rückstand wurde aus Hexan (4 ml) umkristallisiert. Ausbeute: 0,417 g (0,659 mmol; 40 %).

Elementaranalyse (ber. für $C_40H_{41}O_5P$ = 632,70 g/ mol) C 75,95 (75,93); H 6,52 (6,53); P 5,01 (5,00) %.

$^{31}$P-NMR ($CD_2Cl_2$): 140,9 ppm.

$^1$H-NMR ($CD_2Cl_2$): 1,37 (s, 18 H); 3,84 (s, 6 H); 6,51 (d, $^5J_{HH}$= 3,1 Hz, 2 H, $H_{arom}$); 6,89-7,95 (m, br, 15 H, $H_{arom}$)ppm.

$^{13}$C-NMR ($CD_2Cl_2$): 31,1; 35,5; 55,9; 113,0; 114,5; 125,2; 126,5-131,0 (broad, overlapping signals); 133,8 (d,

$J_{cp}$= 4,0 Hz); 141,5 (d, $J_{cp}$= 6,3 Hz); 142,6; 144,8; 156,1 ppm. ESI-TOF/HRMS: m/e 633,27654 $(M+H)^+$.

**Ligand 6 (Vergleichsbeispiel)**

6-([1,1':3',1"-Terphenyl]-2'-yloxy)-4-(*tert*-butyl)-2-methoxybenzo[*d*]naphtho[1,2-*f*][1,3,2]dioxaphosphepin.

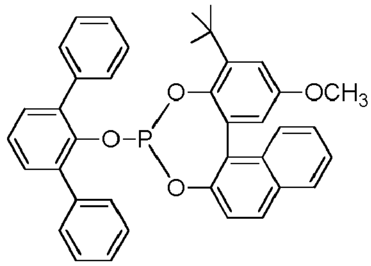

(**6**)

Eine Lösung von 1-(3-(*tert*-Butyl)-2-hydroxy-5-methoxyphenyl)naphthalen-2-ol (0,539 g; 1,673 mmol) in Toluol (14 ml) wurde mit Triethylamin (1,550 g; 15,320 mmol) versetzt und auf 0 °C gekühlt. Zu dieser Mischung wurde eine Lösung von 2,6-Diphenylphenoxydichlorphosphin (0,581 g; 1,673 mmol) in Toluol (3 ml) tropfenweise zugegeben. Die Reaktionsmischung wurde über Nacht gerührt und dann filtriert. Das Filtrat wurde im Vakuum bis zur Trockne eingeengt. Der erhaltene Rückstand wurde in Dichlormethan (3 ml) aufgenommen, die erhaltene Suspension über Kieselgel filtriert und das Filtrat im Vakuum zur Trockne eingeengt. Ausbeute: 0,836 g (1,401 mmol; 86 %). Elementaranalyse (ber. für $C_{39}H_{33}O_4P$ = 596,66 g/ mol) C 78,58 (78,51); H 5,52 (5,57); P 5,28 (5,19) %.
$^{31}$P-NMR ($CD_2Cl_2$): 148,9 ppm.
$^{1}$H-NMR ($CD_2Cl_2$): 16 (s, 9 H); 3,75 (s, 3 H); 6,81 (d, $J_{HH}$ = 3,2 Hz, 1 H, $H_{arom}$); 6,95 (d, $J_{HH}$ = 3,2 Hz, 1 H, $H_{arom}$); 6,98 (d, $J_{HH}$ = 8,8 Hz, 1 H, $H_{arom}$); 7,14-7,19 (m, 6 H, $H_{arom}$); 7,34-7,42 (m, 3 H, $H_{arom}$); 7,44-7,49 (m, 4 H, $H_{arom}$); 7,51-7,56 (m, 2 H); 7,82 (d, $J_{HH}$ = 8,8 Hz, 1 H, $H_{arom}$); 7,92 (m, 1 H, $H_{arom}$); 7,98 (m, 1 H, $H_{arom}$) ppm.
$^{13}$C-NMR ($CD_2Cl_2$): 31,2 (d, $J_{CP}$= 5,7 Hz); 35,3; 55,9; 114,6 (d, $J_{cp}$= 12,3 Hz); 121,7; 125,3; 125,3; 125,8; 126,1; 126,7; 127,5; 128,4; 128,5; 128,7; 129,3; 130,3; 131,0; 132,1; 132,8; 136,5 (d, $J_{cp}$= 3,2 Hz); 138,8; 141,9 (d, $J_{cp}$= 3,8 Hz); 143,9 (d, $J_{cp}$= 3,0 Hz); 145,9 (d, $J_{cp}$= 3,0 Hz); 147,0 (d, $J_{cp}$= 6,0 Hz); 155,4 ppm.
ESI-TOF/HRMS: m/e 597,21901 $(M+H)^+$.

Ligand 7 (Vergleichsbeispiel)

6-(Anthracen-9-yloxy)-2,4,8,10-tetra-*tert*-butyldibenzo[*d,f*][1,3,2]dioxaphosphepin.

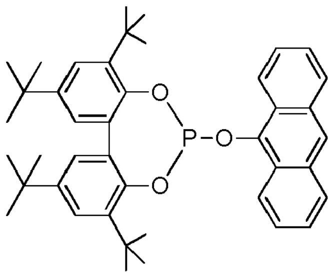

(**7**)

Eine gerührte Suspension von Anthracen-9-ol (0,281 g; 1,45 mmol) in Toluol (7 ml) wurde mit Triethylamin (1,344 g; 13,28 mmol) versetzt und die erhaltene Mischung tropfenweise bei 0 °C zu einer Lösung von 2,4,8,10-Tetra-*tert*-butyl-6-chlorodibenzo [*d,f*][1,3,2]dioxaphosphepin (0,724 g; 1,52 mmol) in Toluol (6 ml) gegeben. Die Reaktionsmischung wurde über Nacht gerührt und filtriert. Das Filtrat wurde im Vakuum bis zur Trockne eingeengt und der erhaltene Rückstand aus Hexan (4 ml) umkristallisiert. Ausbeute: 0,559 g (0,883 mmol; 61 %).
Elementaranalyse (ber. für $C_{42}H_{49}O_3P$ = 632,78 g/ mol) C 79,83 (79,71); H 7,61 (7,81); P 5,01 (4,89) %.
$^{31}$P-NMR ($CD_2Cl_2$): 140,7 ppm.

$^{1}$H-NMR ($CD_2Cl_2$): 1,41 (s, 18 H, $C(CH_3)_3$); 1,50 (s, 18 H, $C(CH_3)_3$); 7,40 (d, $^{5}J_{HH}$= 2,4 Hz, 2 H, $H_{arom}$); 7,43-7,56 (m, 4 H, $H_{arom}$); 7,60 (d, $^{5}J_{HH}$= 2,4 Hz, 2 H, $H_{arom}$); 8,05 (m, 2 H, $H_{arom}$); 8,34 (s, 1 H, $H_{arom}$); 8,38 (m, 2 H, $H_{arom}$) ppm.
$^{13}$C-NMR ($CD_2Cl_2$): 31,2; 31,2; 31,8; 35,1; 35,7; 123,1; 123,7 (d, $J_{cp}$= 5,3 Hz); 124,8; 125,0; 125,8; 126,0; 127,2; 128,4; 132,6; 133,3 (d, $J_{cp}$= 3,7 Hz); 141,0; 143,5; 145,8 (d, $J_{cp}$= 6,2 Hz); 147,7 ppm.
ESI-TOF/HRMS: m/e 633,34934 $(M+H)^+$.

**Durchführung der Katalyseversuche**

Die Hydroformylierung wurde in mit Druckkonstanthaltung, Gasflussmessung, Begasungsrührer und Druckpipette ausgestattete 200 ml-Autoklaven der Fa. Premex Reactor AG, Lengau, Schweiz, durchgeführt. Das als Lösungsmittel verwendete Toluol wurde mit einem Pure Solv MD-7 System gereinigt und unter Argon aufbewahrt. Das als Substrat eingesetzte Substrat n-Octene (Octenisomerengemisch aus 1-Octen: ~3 %; *cis+trans*-2-Octen: 48 %; *cis+trans-3*-Octen: 29%; *cis+trans*-Octen-4: 17 %; gerüstisomere Octene: 3 %) wurde mehrere Stunden über Natrium am Rückfluß erhitzt und unter Argon destilliert.
Für die Versuche wurden im Autoklaven unter Argonatmosphäre Lösungen der Katalysatorvorstufe und des Liganden gemischt. Als Katalysatorvorstufe kam [(acac)Rh(COD)] (Umicore, acac = Acetylacetonat-Anion; COD = 1,5-Cyclooctadien) zum Einsatz. Für Versuche mit einer Konzentration von 100 ppm-m Rhodium wurden 10 ml einer 4,31 millimolaren Lösung in den Autoklaven gegeben. Anschließend wurde die einem Verhältnis L/Rh = 5:1 entsprechende Masse des Liganden in 10 ml Toluol gelöst und zugemischt. Durch Zugabe von weiterem Toluol wurde das Anfangsvolumen der Katalysatorlösung auf 41,0 ml eingestellt. In eine druckfeste Pipette wurde eingefüllt: n-Octene (10,70 g; 95,35 mmol). Der Autoklav wurde mit Synthesegas (Linde; $H_2$ (Qualität 5.0): CO (Qualität 4.7) = 1:1) auf einen Gesamtdruck von 42 bar gebracht und auf 120°C aufgeheizt. Nach Erreichen der Reaktionstemperatur wurde der Synthesegasdruck im Autoklaven auf 48,5 bar erhöht und das Olefingemisch mit einem in der Druckpipette eingestellten Druck von ca. 51,5 bar in den Autoklaven gepresst. Die Reaktion wurde bei konstantem Druck von 20 bar (Nachdruckregler der Fa. Bronkhorst, NL) über 4 h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 5 ml Pentan verdünnt und gaschromatographisch analysiert: HP 5890 Series II plus, PONA, 50 m x 0,2 mm x 0,5 $\mu$m.
Die Ergebnisse sind in der nachfolgenden Tabelle gezeigt.

| Ligand | Ausbeute [%] | Selektivität [%] |
|---|---|---|
| 1* | 98 | 39,3 |
| 2* | 97 | 38,5 |
| 4* | 99 | 30,3 |
| Alkanox 240 | 90 | 28,2 |
| 5 | 29 | 32,1 |
| 6 | 69 | 33,9 |
| 7 | 86 | 31,6 |
| * erfindungsgemäße Verbindungen | | |

$$\text{Selektivität \%} = n / (n + i)$$

Alkanox 240 wurde unter identischen Bedingungen gemessen. Bei Alkanox 240 handelt es sich um einen aus dem Stand der Technik bekannten Triphosphitliganden Tris(2,4-di-tert-butylphenyl)phosphit.
Die Vergleichsverbindungen 5 bis 7 erzielen zwar ähnlich gute n-Selektivitäten wie die erfindungsgemäßen Verbindungen, jedoch zeigen die erfindungsgemäßen Verbindungen deutlich bessere Ausbeuten. Eine hohe Ausbeute ist wichtig, da dadurch mehr Rohstoff in das gewünschte Wertprodukt umgesetzt wird und somit weniger Abfälle produziert werden.

**Patentansprüche**

1. Verbindung gemäß Formel (I)

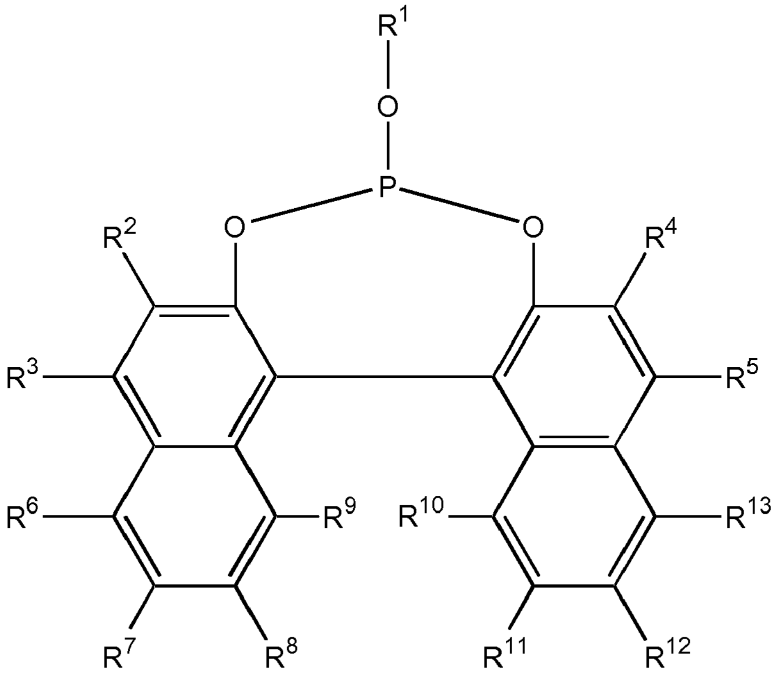

(I)

wobei $R^1$ Anthracenyl oder Diphenylphenyl darstellt; $R^2$ bis $R^{13}$ unabhängig voneinander ausgewählt sein können aus -H, -($C_6$-$C_{20}$)-Aryl, -($C_1$-$C_{12}$)-Alkyl, -($C_3$-$C_{12}$)-Cycloalkyl, -($C_3$-$C_{12}$)-Heterocycloalkyl, -O-($C_1$-$C_{12}$)-Alkyl, -O-($C_3$-$C_{12}$)-Cycloalkyl, -N-[($C_1$-$C_{12}$)-Alkyl]$_2$, -COOH, -OH, -$SO_3H$, -$NH_2$, und Halogen.

2. Verbindung nach Anspruch 1, wobei $R^2$ bis $R^{13}$ unabhängig voneinander ausgewählt sein können aus -H, -($C_1$-$C_{12}$)-Alkyl, und -O-($C_1$-$C_{12}$)-Alkyl.

3. Verbindung nach einem der Ansprüche 1 oder 2, wobei $R^1$ Anthracenyl oder Diphenylphenyl darstellt und $R^2$ bis $R^{13}$ jeweils -H darstellen.

4. Verbindung nach einem der Ansprüche 1 bis 3, welche eine der folgenden Strukturen (1), (2), oder (4) aufweist:

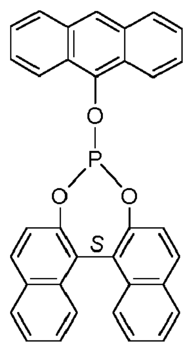

(1)

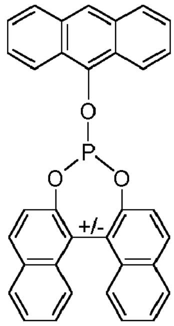

(**2**)

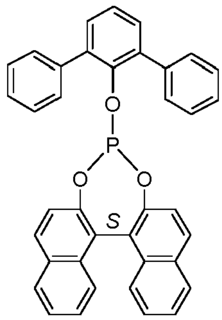

(**4**)

**5.** Komplex umfassend eine Verbindung nach einem der Ansprüche 1 bis 4 und ein Metallatom ausgewählt aus Rh, Ru, Co und Ir.

**6.** Verwendung einer Verbindung nach einem der Ansprüche 1 bis 4 zur Katalyse einer Hydroformylierungsreaktion.

**7.** Verwendung eines Komplexes nach Anspruch 5 zur Katalyse einer Hydroformylierungsreaktion.

**8.** Verfahren zur Herstellung eines Aldehyds, umfassend die Verfahrensschritte:

a) Vorlegen eines Olefins,
b) Zugabe eines Komplexes nach Anspruch 5 oder einer Verbindung nach einem der Ansprüche 1 bis 4 und einer Substanz, welche ein Metallatom ausgewählt aus Rh, Ru, Co und Ir aufweist,
c) Zuführen von Wasserstoff und Kohlenstoffmonoxid,
d) Erwärmen des Reaktionsgemisches, wobei das Olefin zu einem Aldehyd umgesetzt wird.

**9.** Verfahren nach Anspruch 8,
wobei das molare Verhältnis der Verbindung nach einem der Ansprüche 1 bis 9 zu dem Metallatom nach Anspruch 10 im Bereich von 10:1 bis 1:1 liegt.

**Geänderte Patentansprüche gemäss Regel 137(2) EPÜ.**

**1.** Verbindung gemäß Formel (I)

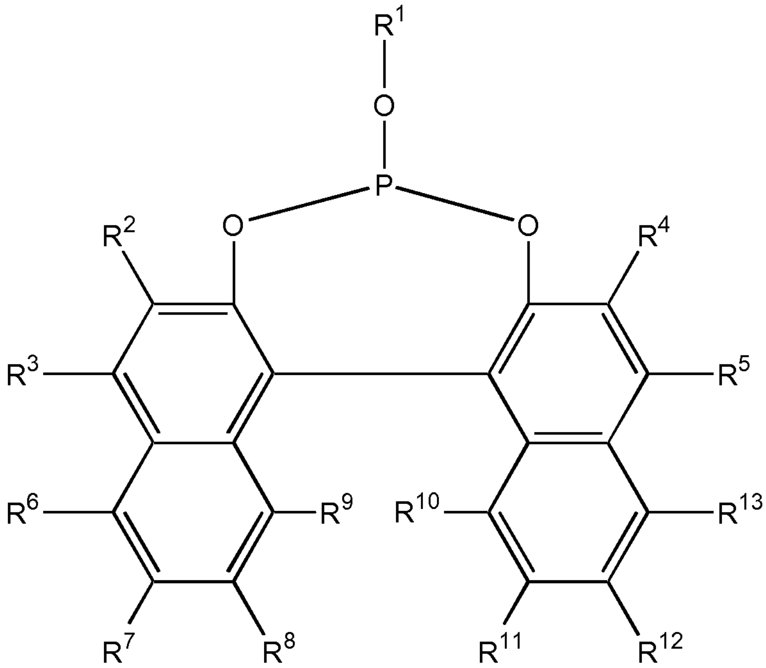

(**I**)

wobei $R^1$ Anthracenyl darstellt;
$R^2$ bis $R^{13}$ unabhängig voneinander ausgewählt sein können aus -H, -($C_6$-$C_{20}$)-Aryl, -($C_1$-$C_{12}$)-Alkyl, -($C_3$-$C_{12}$)-Cycloalkyl, -($C_3$-$C_{12}$)-Heterocycloalkyl, -O-($C_1$-$C_{12}$)-Alkyl, -O-($C_3$-$C_{12}$)-Cycloalkyl, -N-[($C_1$-$C_{12}$)-Alkyl]$_2$, -COOH, -OH, -$SO_3$H, -$NH_2$, und Halogen.

**2.** Verbindung nach Anspruch 1,
wobei $R^2$ bis $R^{13}$ unabhängig voneinander ausgewählt sein können aus -H, -($C_1$-$C_{12}$)-Alkyl, und -O-($C_1$-$C_{12}$)-Alkyl.

**3.** Verbindung nach einem der Ansprüche 1 oder 2,
wobei $R^1$ Anthracenyl darstellt und $R^2$ bis $R^{13}$ jeweils -H darstellen.

**4.** Verbindung nach einem der Ansprüche 1 bis 3,
welche eine der folgenden Strukturen (1) oder (2) aufweist:

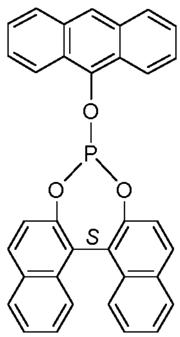

(**1**)

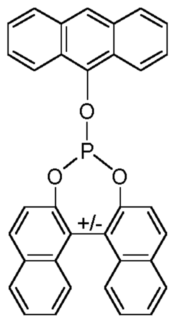

(**2**)

**5.** Komplex umfassend eine Verbindung nach einem der Ansprüche 1 bis 4 und ein Metallatom ausgewählt aus Rh, Ru, Co und Ir.

**6.** Verwendung einer Verbindung nach einem der Ansprüche 1 bis 4 zur Katalyse einer Hydroformylierungsreaktion.

**7.** Verwendung eines Komplexes nach Anspruch 5 zur Katalyse einer Hydroformylierungsreaktion.

**8.** Verfahren zur Herstellung eines Aldehyds, umfassend die Verfahrensschritte:

a) Vorlegen eines Olefins,
b) Zugabe eines Komplexes nach Anspruch 5 oder einer Verbindung nach einem der Ansprüche 1 bis 4 und einer Substanz, welche ein Metallatom ausgewählt aus Rh, Ru, Co und Ir aufweist,
c) Zuführen von Wasserstoff und Kohlenstoffmonoxid,
d) Erwärmen des Reaktionsgemisches, wobei das Olefin zu einem Aldehyd umgesetzt wird.

**9.** Verfahren nach Anspruch 8,
wobei das molare Verhältnis der Verbindung nach einem der Ansprüche 1 bis 9 zu dem Metallatom nach Anspruch 10 im Bereich von 10:1 bis 1:1 liegt.

Europäisches Patentamt
European Patent Office
Office européen des brevets

# EUROPÄISCHER RECHERCHENBERICHT

Nummer der Anmeldung
EP 16 18 6615

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | Manfred T Reetz ET AL: "Why are BINOL-based monophosphites such efficient ligands in Rh-catalyzed asymmetric olefin hydrogenation?", Journal of the American Chemical Society, 27. Juli 2005 (2005-07-27), Seiten 10305-10313, XP055244563, United States DOI: 10.1021/ja052025+ Gefunden im Internet: URL:http://pubs.acs.org/doi/pdf/10.1021/ja052025+ [gefunden am 2016-01-25] * Verbindung 11 * ----- | 1-5 | INV. C07F9/6574 C07C45/50 |
| X | BEDFORD R B ET AL: "Asymmetric styrene dimerisation using mixed palladium-indium catalysts", TETRAHEDRON, ELSEVIER SCIENCE PUBLISHERS, AMSTERDAM, NL, Bd. 61, Nr. 41, 10. Oktober 2005 (2005-10-10), Seiten 9799-9807, XP027861583, ISSN: 0040-4020 [gefunden am 2005-10-10] * Seite 9803; Tabelle 4; Verbindung 18 * ----- | 1-4 | RECHERCHIERTE SACHGEBIETE (IPC) C07F C07C |
| X | US 4 599 206 A (BILLIG ERNST [US] ET AL) 8. Juli 1986 (1986-07-08) * Tabelle 3; Verbindungen 7, 9 * ----- -/-- | 1-9 | |

Der vorliegende Recherchenbericht wurde für alle Patentansprüche erstellt

2

| Recherchenort | Abschlußdatum der Recherche | Prüfer |
|---|---|---|
| München | 12. Oktober 2016 | Duval, Eric |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 16 18 6615

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | JUE XIAO CAI ET AL: "AN IMPROVED METHOD FOR THE RESOLUTION OF 1,1'-BINAPHTHALENE-2,2'-DIOL VIA A PHOSPHITE USING (-)-MENTHOL AS A RESOLVING AGENT", PHOSPHORUS, SULFUR AND SILICON AND THE RELATED ELEMENTS, TAYLOR & FRANCIS INC, US, Bd. 177, Nr. 1, 1. Januar 2002 (2002-01-01), Seiten 189-193, XP008024743, ISSN: 1042-6507, DOI: 10.1080/10426500210215 * Verbindung 3 * ----- | 1-9 | |
| A | THOMAS JERPHAGNON ET AL: "Enantioselective Hydrogenation of[beta]-Acylamino Acrylates Catalyzed by Rhodium(I)-Monophosphite Complexes", ADVANCED SYNTHESIS & CATALYSIS, Bd. 346, Nr. 1, 1. Januar 2004 (2004-01-01), Seiten 33-36, XP055244591, DE ISSN: 1615-4150, DOI: 10.1002/adsc.200303144 * Verbindungen 1a, 1b * ----- -/-- | 1-9 | RECHERCHIERTE SACHGEBIETE (IPC) |

Der vorliegende Recherchenbericht wurde für alle Patentansprüche erstellt

2

| Recherchenort | Abschlußdatum der Recherche | Prüfer |
|---|---|---|
| München | 12. Oktober 2016 | Duval, Eric |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 16 18 6615

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | MAJI P ET AL: "Studies directed toward the synthesis of chiral tungsten and molybdenum carbonyl complexes", JOURNAL OF ORGANOMETALLIC CHEMISTRY, ELSEVIER-SEQUOIA S.A. LAUSANNE, CH, Bd. 693, Nr. 10, 1. Mai 2008 (2008-05-01), Seiten 1841-1849, XP022607994, ISSN: 0022-328X, DOI: 10.1016/J.JORGANCHEM.2008.02.008 [gefunden am 2008-02-15] * Scheme 1; Verbindungen L-c * ----- | 1-9 | |

RECHERCHIERTE SACHGEBIETE (IPC)

Der vorliegende Recherchenbericht wurde für alle Patentansprüche erstellt

2

| Recherchenort | Abschlußdatum der Recherche | Prüfer |
|---|---|---|
| München | 12. Oktober 2016 | Duval, Eric |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 16 18 6615

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-10-2016

| Im Recherchenbericht angeführtes Patentdokument | Datum der Veröffentlichung | Mitglied(er) der Patentfamilie | Datum der Veröffentlichung |
|---|---|---|---|
| US 4599206 A | 08-07-1986 | AT 83230 T | 15-12-1992 |
| | | AU 579144 B2 | 17-11-1988 |
| | | AU 617362 B2 | 28-11-1991 |
| | | AU 2278888 A | 19-01-1989 |
| | | AU 3997085 A | 10-09-1985 |
| | | BR 8505278 A | 18-02-1986 |
| | | CA 1262915 A1 | 14-11-1989 |
| | | CS 8501091 A2 | 12-02-1990 |
| | | DE 3586878 D1 | 21-01-1993 |
| | | DE 3586878 T2 | 24-06-1993 |
| | | DK 473485 A | 11-12-1985 |
| | | EP 0155508 A1 | 25-09-1985 |
| | | ES 8609185 A1 | 16-12-1986 |
| | | ES 8701140 A1 | 16-02-1987 |
| | | ES 8706480 A1 | 16-09-1987 |
| | | FI 854059 A | 17-10-1985 |
| | | HU 202176 B | 28-02-1991 |
| | | JP H0645633 B2 | 15-06-1994 |
| | | JP S61501268 A | 26-06-1986 |
| | | MX 164254 B | 27-07-1992 |
| | | NO 165915 B | 21-01-1991 |
| | | PL 151491 B1 | 28-09-1990 |
| | | PL 251973 A1 | 29-07-1986 |
| | | RO 92321 B | 01-09-1987 |
| | | SG 32193 G | 21-05-1993 |
| | | US 4599206 A | 08-07-1986 |
| | | WO 8503702 A1 | 29-08-1985 |
| | | YU 22585 A | 29-02-1988 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0155508 A1


### In der Beschreibung aufgeführte Nicht-Patentliteratur


- **B. CORNILS ; W. A. HERRMANN.** Applied Homogeneous Catalysis with Organometallic Compounds. VCH, 1996, vol. 1 & 2
- **R. FRANKE ; D. SELENT ; A. BÖRNER.** Applied Hydroformylation. *Chem. Rev.,* 2012
- **VAN LEEUWEN.** *Journal of Catalysis,* 2013, vol. 298, 198-205
- Applied Hydroformylation. **R. FRANKE ; D. SELENT ; A. BÖRNER.** Chem. Rev. 2012, vol. 112, 5681
- **R. FRANKE ; D. SELENT ; A. BÖRNER.** Applied Hydroformylation. *Chem. Rev.,* 2012, 5688
- **P. W. N. M. VAN LEEUWEN.** Rhodium Catalyzed Hydroformylation. Kluwer, 2000, S. 48 ff, , S.233 ff
- **K.D. WIESE ; D. OBST.** Top. Organomet. Chem. Springer Verlag, 2006, vol. 18, 6 ff